# EUROPÄISCHE PATENTSCHRIFT

(11) **EP 0 599 215 B1**
(45) Veröffentlichungstag und Bekanntmachung des Hinweises auf die Patenterteilung: **30.11.1994**
(21) Anmeldenummer: 93118648.0
(22) Anmeldetag: 19.11.1993
(51) Int. Cl.: C07C 45/90, C07C 49/88

(54) **Verfahren zur Isolierung von reinem Diketen unter Rückgewinnung von Wertstoffen**
Method for the isolation of pure diketene and recovery of valuable substances
Procédé d'isolement de dicétène et récupération de substances valables

(30) Priorität: 20.11.1992 DE 4239117
(43) Veröffentlichungstag der Anmeldung: 01.06.1994
(73) Patentinhaber: Wacker-Chemie GmbH, 81737 München (DE)
(72) Erfinder: Künstle, Gerhard, Dr., D-84489 Burghausen (DE); Maier, Alois, D-84489 Burghausen (DE)

(56) Entgegenhaltungen:
- DATABASE WPI Week 8638, Derwent Publications Ltd., London, GB; AN 86-248751; & JP-A-61 176 581

## Beschreibung

Die Erfindung betrifft ein Verfahren zur Isolierung von reinem Diketen unter Rückgewinnung von Wertstoffen durch kontinuierliche, zweistufige Destillation von Rohdiketen unter vermindertem Druck und kontinuierlicher Umsetzung des dabei anfallenden Destillationsrückstands mit Essigsäure.

Bei der industriellen Dimerisierung von technischem Keten, beispielsweise nach der DE-OS 2301655 (US-A 4001332), wird ein 82- bis 87-%iges Rohdiketen erhalten, das an Verunreinigungen neben wenig gelöstem Keten, Aceton und Essigsäure noch 5 bis 7 % Essigsäureanhydrid sowie 8 bis 10 % oligo- bis höherpolymere Ketene enthält, die durch die allgemeine Formel (CH₂=C=O)ₙ₊₁, wobei n > 1 ist, beschrieben werden können. Dieses Rohdiketen ist als Ausgangsstoff für zahlreiche Synthesen ungeeignet. Es muß vor seinem weiteren Einsatz gereinigt werden, was zweckmäßigerweise durch Destillation geschehen kann. Wird dabei auf bekannte Weise verfahren, so müssen wegen der hohen Reaktivität des Diketens Produktzersetzung und Polymerisation in Kauf genommen werden, worunter die Wirtschaftlichkeit und die Effektivität des Reinigungsverfahrens, unter Beeinträchtigung der Qualität des erhaltenen Reindiketens, leiden. Darüberhinaus ist die Destillation des Rohdiketens, insbesonders durch das zu 4 bis 5 % stets enthaltene Ketentrimere, das sehr unbeständig ist, mit einem hohen Sicherheitsrisiko behaftet.

Als verfahrens- und sicherheitstechnisch besonders schwierig erweist sich bei der technischen Durchführung der Rohdiketen-Destillation die Abtrennung und weitere Handhabung der verbleibenden höherpolymeren Ketene, die in Form eines unter Normalbedingungen zähflüssigen bis festen - je nach Restgehalt an Diketen und Essigsäureanhydrid - instabilen, zur Spontanzersetzung neigenden, Rückstands anfallen. Daher wird in der Praxis reines Diketen aus dem Rohdiketen nicht quantitativ, sondern nur teilweise, in der Regel etwa zu 50 %, bezogen auf den Ausgangsgehalt, durch Destillation isoliert und das dabei verbleibende, nun noch mehr Nebenprodukte enthaltende Rohdiketen der weiteren Verwertung mit all den damit verbundenen Nachteilen zugeführt. Darüberhinaus kann dieser instabile Rückstand keiner Wiederverwertungsmaßnahme zugeführt werden, sondern muß umgehend und fortlaufend entsorgt werden, beispielsweise durch Verbrennung, woraus sich ein spürbarer Wertstoffverlust, bezogen auf Keten, sowie eine unerwünschte Kopplung an ein dauernd verfügbares Rückstandsentsorgungssystem ergeben.

Es wurde schon versucht, bei der Dimerisation von Keten den Anteil an polymeren Ketenen durch Zugabe von Inhibitoren, beispielsweise Schwefeldioxid, zu verringern (EP-A 0377438 = US-A 4999438). Dadurch kann das Problem zwar verbessert, jedoch nicht gelöst werden. Es verbleibt in jedem Fall ein Restgehalt an höherpolymeren Ketenen von 8 bis 10 %. Außerdem wird durch derartige Zusätze die Stabilität des Rohdiketens bei der destillativen Reinigung eher verringert, erkennbar an geringeren Destillationsausbeuten bzw. vermehrter Bildung von reaktivem Rückstand.

Der andere Lösungsweg, aus dem Rohdiketen nur einen Teil des Diketens destillativ in reiner Form zu gewinnen und den verbleibenden Rest geeigneten Folgereaktionen zuzuführen, beispielsweise der Herstellung verschiedener Acetessigester, löst nur den sicherheitstechnischen Aspekt des Problems. Neben den Nachteilen einer Koppelproduktion müssen in diesem Fall bei den Folgesynthesen andere schwerwiegende Nachteile in Kauf genommen werden, die zu hohem Stoffverbrauch, hohen Fertigungskosten und Qualitätsproblemen führen, infolge der Bildung zahlreicher Nebenprodukte, die aus dem Zielprodukt wieder entfernt werden müssen.

Gemäß der EP-A 0287894 (US-A 4808735) versuchte man daher, das Sicherheitsrisiko bei der Herstellung und Destillation von Rohdiketen durch Zugabe von Verbindungen mit funktionellen Hydroxylgruppen, beispielsweise Wasser, Alkoholen oder Carbonsäuren, die mit dem trimeren Keten zu thermostabilen Butancarbonsäurederivaten reagieren, zu verringern. Da jedoch mit diesen Zusätzen auch das Diketen analoge Reaktionen eingeht, ist der gewollte Abbau bezogen auf Triketen nur unvollständig oder es muß ein hoher Überschuß an diesen Zusatzstoffen angewandt werden, wodurch sich diese Maßnahme, aufgrund der Nebenreaktionen mit Diketen, als unbrauchbar erweist.

Die gleichen Nachteile weisen die Verfahren gemäß CA-A 846162 und CA-A 850145 auf, bei denen das Rohdiketen zur Entfernung von Acetanhydrid-Beiprodukt mit Wasser umgesetzt wird und anschließend mittels Extraktion und/oder Destillation aufgearbeitet wird.

Es bestand daher die Aufgabe ein Verfahren zur gefahrlosen Isolierung von reinem Diketen unter Vermeidung von Produktverlusten durch Zersetzung und Polymerisation, aus einem auf bekannte Weise zugänglichen diketenhaltigen Reaktionsgemisch (Rohdiketen), das neben geringen Mengen Keten, Aceton und Essigsäure noch Essigsäureanhydrid und polymere Ketene enthält, zu entwickeln.

Gegenstand der Erfindung ist ein Verfahren zur Isolierung von reinem Diketen unter Rückgewinnung von Wertstoffen durch kontinuierliche, zweistufige Destillation von Rohdiketen unter vermindertem Druck und kontinuierlicher Umsetzung des dabei anfallenden Destillationsrückstands mit Essigsäure, wobei man
a) in der ersten Destillationsstufe das Rohdiketen einem Dünnschichtverdampfer zuführt, bei vermindertem Druck verdampft und die Dampfphase einer Destillationskolonne zuführt, über deren Kolonnenkopf man reines Diketen abzieht und den, ein Gemisch aus Diketen und Essigsäureanhydrid enthaltenden, Kolonnensumpf mit dem Sumpfprodukt des Dünnschichtverdampfers vereinigt, und
b) zum Gemisch der Sumpfprodukte von Dünnschichtverdampfer bzw. Destillationskolonne der ersten Destillationsstufe noch Kondensat, welches bei der Produktion von Keten bzw. dessen Weiterverarbeitung anfällt, eingeschleust wird oder reines Essigsäureanhydrid, welches als Sumpfprodukt bei der Destillation in der zweiten Destillationsstufe anfällt dem Sumpfproduktgemisch der ersten Destillationsstufe aufgegeben und im Kreis geführt wird, und man
c) in der zweiten Destillationstufe die vereinigten Sumpfprodukte einem weiteren Dünnschichtverdampfer zuführt, bei vermindertem Druck verdampft und die Dampfphase in eine Destillationskolonne überführt, über deren Kolonnenkopf man ein Diketen und Essigsäureanhydrid enthaltendes Gemisch abzieht, welches man in die Destillationskolonne der ersten Destillationsstufe zurückführt, während man das Sumpfprodukt in Form von reinem Essigsäureanhydrid ausschleust oder im Kreis führt, und man
d) den Sumpfaustrag aus dem Dünnschichtverdampfer der zweiten Destillationsstufe mit Essigsäure versetzt, einem Reaktor zuführt und nach erfolgter Umsetzung das Reaktionsprodukt in einem Dünnschichtverdampfer auftrennt, wobei als Brüden wiederverwertbare Essigsäure, welche Aceton und gegebenenfalls Essigsäureanhydrid enthält, anfällt, während als Sumpfprodukt ein reaktionsinerter Rückstand in flüssiger Form abgezogen wird.

Das mit der erfindungsgemäßen Verfahrensweise zu reinigende diketenhaltige Reaktionsgemisch (Rohdiketen) enthält im allgemeinen neben geringen Mengen Keten, Aceton und Essigsäure noch 5 bis 7 % Essigsäureanhydrid und 8 bis 10 % polymere Ketene. Das Reaktionsgemisch fällt bei der Herstellung von Diketen nach allgemein bekannten Verfahren, beispielsweise durch Dimerisierung von Keten, welches nach thermischer Wasserabspaltung aus Essigsäure erhalten wird, als Rohprodukt, welches einer weiteren Reinigung bedarf, an.

Zur Reinigung wird das Rohdiketen zunächst dem Dünnschichtverdampfer der ersten Destillationsstufe zugeführt, welcher mit vermindertem Druck und erhöhter Temperatur betrieben wird. Vorzugsweise wird der Dünnschichtverdampfer bei einem Druck von 50 bis 100 mbar und einer Temperatur von höchstens 95°C, vorzugsweise von 60 bis 85°C betrieben. Als Dünnschichtverdampfer geeignet sind die im Handel erhältlichen Systeme, welche aus einem zylindrischen, über einen Heizmantel beheizten, Verdampfungsraum bestehen, der einen mit Wischerflächen ausgerüsteten Rotationskörper enthält.

Mit der erfindungsgemäßen Verfahrensweise fällt bei der Dünnschichtverdampfung ein Sumpfprodukt an, welches polymere Ketene, Diketen und Essigsäureanhydrid enthält. Das Kopfprodukt enthält Diketen und Essigsäureanhydrid. Zur Isolierung des reinen Diketens wird das Kopfprodukt in die Destillationskolonne der ersten Destillationsstufe überführt. Geeignet sind beispielsweise normale Füllkörperkolonnen, die aus mehreren Kolonnenschüssen nebst Verteilerböden bestehen und mit Pallringen aus V4A-Stahl gefüllt sind.

Die destillative Auftrennung erfolgt vorzugsweise im gleichen Temperatur- und Druckbereich wie beim Dünnschichtverdampfer der ersten Destillationsstufe. Als Kopfprodukt erhält man Rein-Diketen mit einem Reinheitsgrad von mindestens 99 %, welches abgezogen und dessen weiterer Verwendung zugeführt wird. Mit der erfindungsgemäßen Verfahrensweise werden mindestens 99 % des im Rohdiketen enthaltenen Diketens als Kopfprodukt in der ersten Destillationsstufe isoliert.

Das, im wesentlichen Essigsäureanhydrid mit Diketen-Anteilen enthaltende, Sumpfprodukt der Destillationskolonne wird mit dem, im wesentlichen polymere Ketene enthaltendem, Sumpfprodukt des Dünnschichtverdampfers vereinigt und der zweiten Destillationsstufe zugeführt. Um ein sicheres Arbeiten bei möglichst vollständiger Isolierung des Diketens zu erreichen, hat es sich als zweckmäßig erwiesen, dem Gemisch der Sumpfprodukte aus dem Dünnschichtverdampfer und der Destillationskolonne der ersten Destillationsstufe zusätzliches Essigsäureanhydrid aufzugeben, indem beispielsweise das als Sumpfprodukt bei der Destillation in der zweiten Destillationsstufe anfallende Essigsäureanhydrid im erforderlichen Maß im Kreislauf geführt wird. Eine bevorzugte Ausführungsform besteht darin, das Essigsäureanhydrid nicht im Kreis zu fahren, sondern in Form von Kondensat, welches bei der Produktion von Keten bzw. dessen Weiterverarbeitung anfällt und polymere Ketene, Essigsäureanhydrid und Diketen enthält, einzuschleusen. Besonders bevorzugt ist ein Gewichtsverhältnis von Kondensat zu Sumpfprodukt Dünnschichtverdampfer zu Sumpfprodukt Destillationskolonne von 0.1 bis 0.2 : 1 : 1. Essigsäureanhydrid wird erfindungsgemäß nur dann im Kreis geführt, wenn kein bzw. zu wenig Kondensat zur Verfügung steht.

Der apparative Aufbau der zweiten Destillationsstufe entspricht dem der ersten Destillationsstufe. Die Betriebsbedingungen bezüglich Druck und Temperatur von Dünnschichtverdampfer und Destillationskolonne entsprechen denen der ersten Destillationsstufe. Im Dünnschichtverdampfer der zweiten Destillationsstufe werden die Sumpfprodukte der ersten Destillationstufe in ein Essigsäureanhydrid/Diketen-Gemisch als Kopfprodukt und polymere Ketene als Sumpfablauf aufgetrennt. Das Kopfprodukt wird in die nachfolgende Kolonne überführt. Als Sumpfprodukt erhält man in der Kolonne reines Essigsäureanhydrid, welches im Kreis geführt oder ausgeschleust und einer weiteren Verwendung zugeführt werden kann. Das als Kopfprodukt anfallende essigsäureanhydridhaltige Diketen wird in die Kolonne der ersten Destillationsstufe zurückgeführt.

Der weitgehend diketenfreie Sumpfablauf des Dünnschichtverdampfers der zweiten Destillationsstufe wird im nächsten Schritt des erfindungsgemäßen Verfahrens der Phlegmatisierung mit Essigsäure unterzogen. In einer bevorzugten Ausführungsform wird die Dünnschichtverdampfung in der zweiten Destillationsstufe so geführt, daß der Sumpfablauf mindestens einen Essigsäureanhydridgehalt von 15 Gew% aufweist und der Sumpfablauf in dieser Form umgehend und kontinuierlich der Phlegmatisierung mit Essigsäure unterzogen wird.

Die Phlegmatisierung wird in einem, den beiden Destillationsstufen nachgeschaltetem, Reaktor durchgeführt, der vorzugsweise mit Rührwerk und Rückflußeinrichtung ausgestattet ist. Die Umsetzung mit Essigsäure erfolgt vorzugsweise unter Normaldruck und bei einer Temperatur von 100 bis 150°C. Als Phlegmatisierungsmittel wird Essigsäure in konzentrierter oder verdünnter Form verwendet; vorzugsweise mit einer Konzentration von 50 bis 99.5 Gew%, besonders bevorzugt von 65 bis 95 Gew%. Die Verdünnung der Essigsäure sollte dabei so bemessen sein, daß im phlegmatisierten Rückstand kein Wasser vorhanden ist.

Das Mengenverhältnis von Sumpfaustrag aus der zweiten Destillationsstufe zu Essigsäure wird zweckmäßigerweise so bemessen, daß eine vollständige Phlegmatisierung des Rückstands unter Einsatz möglichst geringer Mengen Essigsäure in möglichst kurzer Zeit erreicht wird. Erfahrungsgemäß erweisen sich dabei 0.3 bis 2.0, vorzugsweise 0.6 bis 1.5 Gew.teile Essigsäure je 1 Gew.teil Rückstand sowie Verweilzeiten von 0.2 bis 8.0, vorzugsweise 2.0 bis 4.0 Stunden, je nach apparativer Beschaffenheit des Reaktors als ausreichend.

Bei der erfindungsgemäßen Umsetzung mit Essigsäure unter Rückfluß wird gegebenenfalls noch vorhandenes Diketen unter Bildung von Aceton und Kohlendioxid zersetzt, während die polymeren Ketene, insbesondere unter Bildung von Essigsäure bzw. Essigsäureanhydrid, teilweise abgebaut werden. Zur Abtrennung dieser bei der Phlegmatisierung aus dem unbrauchbaren Rückstand entstehenden, flüchtigen Wertstoffe wie Essigsäure und Essigsäureanhydrid, die noch wenig Aceton, Isopropenylacetat und Acetylaceton enthalten können, wird der nach der Phlegmatisierung nun reaktionsinerte Rückstand in einen Dünnschichtverdampfer überführt.

Die Dünnschichtverdampfung wird bei Normaldruck oder leichtem Vakuum, vorzugsweise bei 400 bis 500 mbar, und einer Temperatur von 100 bis 180°C durchgeführt. Als Kopfprodukte werden die flüchtigen Wertstoffe unter der Maßgabe abgetrennt, daß das Sumpfprodukt in flüssiger, unter Normalbedingungen noch förderbarer Form, ausgetragen werden kann.

Die Figur zeigt eine vorteilhafte Ausführungsform einer Vorrichtung zur Durchführung des Verfahrens:
Über Leitung 1 wird dem Dünnschichtverdampfer 2 Rohdiketen zugeführt und im Vakuum verdampft. Über Leitung 3 gelangt das Diketen und Essigsäureanhydrid enthaltende Kopfprodukt in die Kolonne 4, aus der über Leitung 5 als Kopfprodukt reines Diketen ausgeschleust wird und über Leitung 6 als Sumpfaustrag ein Gemisch aus Diketen und Essigsäureanhydrid abgezogen wird. Letzteres wird gemeinsam mit dem über Leitung 7 aus dem Dünnschichtverdampfer 2 anfallenden Sumpfaustrag über Leitung 9 dem Dünnschichtverdampfer 10 der zweiten Destillationsstufe zugeführt. Über Leitung 8 kann gegebenenfalls Kondensat aus der Ketenproduktion eingeschleust werden. Über Leitung 11 kann Essigsäureanhydrid im Kreislauf geführt werden. Vom Dünnschichtverdampfer 10 wird über Leitung 12 ein Essigsäureanhydrid/Diketen-Gemisch der Kolonne 13 zugeführt. Das Kopfprodukt der Kolonne 13 wird über Leitung 14 der Kolonne 4 zugeführt; der Sumpfablauf über Leitung 15 ausgeschleust. Der Sumpfablauf des Dünnschichtverdampfers 10 wird über Leitung 16 dem Reaktor 17 zugeführt. Zur Zuführung von Essigsäure in den Reaktor 17 dient Leitung 18. Über Leitung 19 kann bei der Phlegmatisierung entstehendes Kohlendioxid entweichen. Das reaktionsinerte Umsetzungsgemisch wird über Leitung 20 dem Dünnschichtverdampfer 21 zugeführt, wobei die flüchtigen Wertstoffe über Leitung 22 abgezogen werden, während der Rückstand über Leitung 23 ausgeschleust wird.

Mit der vorliegenden Erfindung wird ein wirtschaftliches Verfahren zur gefahrlosen und weitgehend quantitativen Isolierung von reinem (mindestens 99-%igem) Diketen aus Rohdiketen unter besonderer Berücksichtigung des Abfallvermeidungs- und Verwertungsprinzips zur Verfügung gestellt. Neben der Aufarbeitung von Kondensat, das bei der Herstellung und Weiterverarbeitung von Keten stets anfällt und bislang einen eigenen Aufarbeitungsprozeß bei geringerer Rückgewinnungsquote erforderte, kann in der bevorzugten Ausführungsform des erfindungsgemäßen Verfahrens, durch die Zuführung von Kondensat aus der Keten-Produktion bzw. -Aufarbeitung, der bei der Diketenabreicherung entstehende Destillationsrückstand, der zur Spontanzersetzung neigt, während der weitgehend quantitativen Diketenabtrennung stabil gehalten werden und letztlich gefahrlos phlegmatisiert werden.

Überraschenderweise werden mit der erfindungsgemäßen Verfahrensweise, je nach Zusammensetzung des Rückstands aus dem Dünnschichtverdampfer der zweiten Destillationsstufe, mindestens 25 bis 35 Gew% der im Rückstand angereicherten polymeren Ketene der allgemeinen Formel (CH₂=C=O)ₙ₊₁, mit n > 1, in wiederverwertbare Produkte, insbesondere wiederverwertbares Essigsäureanhydrid bzw. Essigsäure umgewandelt, je nachdem ob konzentrierte Essigsäure oder verdünnte Essigsäure zur Phlegmatisierung eingesetzt wird.

Die nachfolgenden Beispiele dienen zur weiteren Erläuterung der Erfindung:

### Beispiel 1:

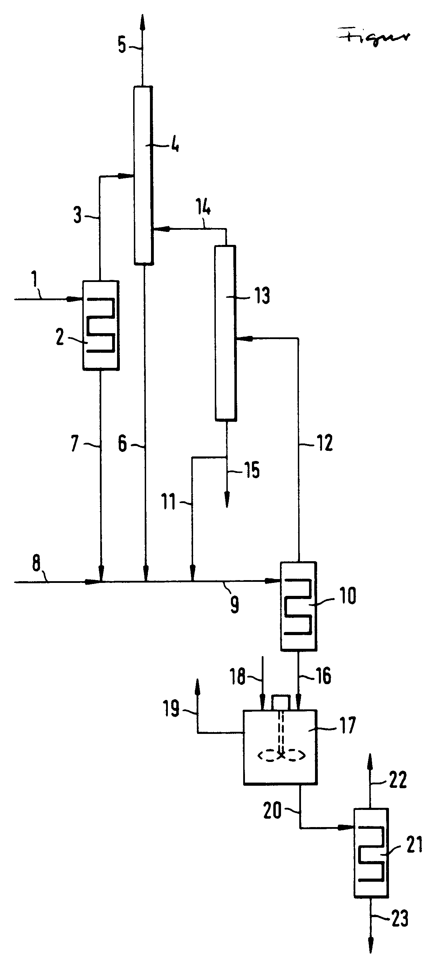

In einer Apparatur gemäß Figur 1 wurden dem Dünnschichtverdampfer 2 stündlich 2016 Gew.teile Rohdiketen der folgenden Zusammensetzung:
83.4 Gew% Diketen
6.9 Gew% Essigsäureanhydrid
9.5 Gew% polymere Ketene
0.2 Gew% Aceton/Essigsäure
zugeführt und dort bei 65°C/52 mbar in essigsäureanhydridhaltiges Diketen als Kopfprodukt und polymere Ketene, Diketen und Essigsäureanhydrid enthaltenden Sumpfablauf getrennt. Das Kopfprodukt des Dünnschichtverdampfers 2 wurde der Kolonne 4, dessen Sumpfablauf gemeinsam mit dem Sumpfablauf aus Kolonne 4 wurde dem Dünnschichtverdampfer 10, zugeführt. Außerdem wurden letzterem über Leitung 8/9 stündlich 31 Gew.teile Kondensat aus der Ketenherstellung, zusammengesetzt aus Essigsäureanhydrid mit 1 Gew% polymeren Ketenen, zugeführt.
Im Dünnschichtverdampfer 10 wurde, unter analogen Betriebsbedingungen wie im Dünnschichtverdampfer 2, in ein diketen/essigsäureanhydridhaltiges Kopfprodukt und in einen, die polymeren Ketene enthaltenden Sumpfablauf, getrennt. Das Kopfprodukt wurde über Leitung 12 der Kolonne 13 zugeführt und dort in Essigsäureanhydrid und Diketen getrennt. Während über Leitung 15 stündlich 94 Gew.teile reines Essigsäureanhydrid ausgeschleust wurden, wurde über Leitung 14 der Kolonne 4 essigsäureanhydridhaltiges Diketen zugeführt und über deren Kopf stündlich 1675 Gew.teile 99.6 %-iges Diketen, das neben 0.2 Gew% Essigsäureanhydrid noch 0.1 Gew% Aceton enthielt, fortlaufend abgezogen
Über Leitung 16 wurden stündlich 276 Gew.teile Sumpfablauf aus dem Dünnschichtverdampfer 10 dem Reaktor 17 zugeführt, dem gleichzeitig über Leitung 18 stündlich 200 Gew.teile 97 %-ige Essigsäure zugeführt wurden. Im Reaktor 17 wurden unter Rühren bei 118°C/Normaldruck die wenig Diketen und 25 Gew% Essigsäureanhydrid enthaltenden polymeren Ketene bei einer durchschnittlichen Verweilzeit von 4 Stunden vollständig phlegmatisiert, wobei 27 Gew% der polymeren Ketene insbesondere zu Essigsäureanhydrid bzw. Essigsäure abgebaut wurden und das Diketen vorwiegend zu Kohlendioxid und Aceton zersetzt wurde.
Gebildetes Kohlendioxid entwich über Leitung 19, das reaktionsinerte Phlegmatisierungsgemisch wurde über Leitung 20 dem Dünnschichtverdampfer 21 zugeführt. Dort wurde unter der Maßgabe, daß der Sumpfaustrag bei Normalbedingungen in flüssiger Form anfallen sollte, der nun reaktionsinerte Rückstand von den flüchtigen Bestandteilen bei 120°C/400 mbar getrennt, wobei über Leitung 23 stündlich 149 Gew.teile Sumpfaustrag mit 3.5 Gew% Essigsäureanhydrid und 96.5 Gew% polymeren Ketenen erhalten wurde. Als Brüden fielen über Leitung 22 stündlich 322 Gew.teile eines Gemisches aus 45 Gew% Essigsäure, 53.1 Gew% Essigsäureanhydrid und 1.9 Gew% Aceton an.

### Beispiel 2:

Es wurde wie in Beispiel 1 verfahren, mit dem Unterschied, daß nicht über Leitung 8 Kondensat zugegeben wurde, sondern über Leitung 11 stündlich 30 Gew.teile reines Essigsäureanhydrid im Kreislauf geführt wurden. Außerdem wurden über Leitung 18 stündlich 150 Gew.teile 90 %-ige Essigsäure eingesetzt. In Reaktor 17 wurden 30 Gew.% der polymeren Ketene zu Essigsäureanhydrid bzw. Essigsäure abgebaut. Über Leitung 22 erhielt man stündlich 280 Gew.teile Brüden mit 2.0 Gew% Aceton, 49.3 Gew% Essigsäure und 48.6 Gew% Essigsäureanhydrid. Über Leitung 23 fielen stündlich 141 Gew.teile reaktionsinerter Rückstand an, der neben 2.1 Gew% Essigsäureanhydrid noch 97.9 Gew% polymere Ketene enthielt.

### Beispiel 3:

Es wurde wie in Beispiel 1 verfahren, mit dem Unterschied, daß über Leitung 8/9 stündlich 62 Gew.teile Kondensat zugeführt wurden. Außerdem wurde der Dünnschichtverdampfer 10 so beheizt, daß bei einem Vakuum von 50 mbar die Sumpfablauftemperatur 95°C betrug. Zudem wurden über Leitung 18 stündlich 146 Gew.teile 68.5 %-ige Essigsäure eingesetzt. Der im Reaktor 17 stattfindende Abbau an polymeren Ketenen betrug 34.8 Gew%, wobei über Leitung 20 stündlich 435 Gew.teile eines Reaktionsgemisches erhalten wurden, das neben 29.7 Gew% polymeren Ketenen in reaktionsinerter Form noch 70.1 Gew% Essigsäure und 0.1 Gew% Aceton enthielt. Dieses Reaktionsgemisch wurde im Dünnschichtverdampfer 21 bei einer Heizflächentemperatur von 180°C und einem Vakuum von 400 mbar in 301 Gew.teile/Stunde 99.7 %-ige, wenig Aceton enthaltende, Essigsäure als Brüden und in 134 Gew.teile/Stunde Rückstand mit einem Essigsäuregehalt von 3.7 Gew% getrennt.

## Patentansprüche

1. Verfahren zur Isolierung von reinem Diketen unter Rückgewinnung von Wertstoffen durch kontinuierliche, zweistufige Destillation von Rohdiketen unter vermindertem Druck und kontinuierlicher Umsetzung des dabei anfallenden Destillationsrückstands mit Essigsäure, wobei man
a) in der ersten Destillationsstufe das Rohdiketen einem Dünnschichtverdampfer zuführt, bei vermindertem Druck verdampft und die Dampfphase einer Destillationskolonne zuführt, über deren Kolonnenkopf man reines Diketen abzieht und den, ein Gemisch aus Diketen und Essigsäureanhydrid enthaltenden, Kolonnensumpf mit dem Sumpfprodukt des Dünnschichtverdampfers vereinigt, und
b) zum Gemisch der Sumpfprodukte von Dünnschichtverdampfer bzw. Destillationskolonne der ersten Destillationsstufe noch Kondensat, welches bei der Produktion von Keten bzw. dessen Weiterverarbeitung anfällt, eingeschleust wird oder reines Essigsäureanhydrid, welches als Sumpfprodukt bei der Destillation in der zweiten Destillationsstufe anfällt dem Sumpfproduktgemisch der ersten Destillationsstufe aufgegeben und im Kreis geführt wird, und man
c) in der zweiten Destillationstufe die vereinigten Sumpfprodukte einem weiteren Dünnschichtverdampfer zuführt, bei vermindertem Druck verdampft und die Dampfphase in eine Destillationskolonne überführt, über deren Kolonnenkopf man ein Diketen und Essigsäureanhydrid enthaltendes Gemisch abzieht, welches man in die Destillationskolonne der ersten Destillationsstufe zurückführt, während man das Sumpfprodukt in Form von reinem Essigsäureanhydrid ausschleust oder im Kreis führt, und man
d) den Sumpfaustrag aus dem Dünnschichtverdampfer der zweiten Destillationsstufe mit Essigsäure versetzt, einem Reaktor zuführt und nach erfolgter Umsetzung das Reaktionsprodukt in einem Dünnschichtverdampfer auftrennt, wobei als Brüden wiederverwertbare Essigsäure, welche Aceton und gegebenenfalls Essigsäureanhydrid enthält, anfällt, während als Sumpfprodukt ein reaktionsinerter Rückstand in flüssiger Form abgezogen wird.

2. Verfahren nach Anspruch 1, dadurch gekennzeichnet, daß die Dünnschichtverdampfer und die Kolonnen der beiden Destillationsstufen bei einem Druck von 50 bis 100 mbar und einer Temperatur von 60 bis 85°C betrieben werden.

3. Verfahren nach Anspruch 1 bis 2, dadurch gekennzeichnet, daß die Dünnschichtverdampfung in der zweiten Destillationsstufe so geführt wird, daß der Sumpfablauf mindestens einen Essigsäureanhydridgehalt von 15 Gew% aufweist und der Sumpfablauf in dieser Form umgehend und kontinuierlich der Phlegmatisierung mit Essigsäure unterzogen wird.

4. Verfahren nach Anspruch 1 bis 3, dadurch gekennzeichnet, daß die Umsetzung mit Essigsäure unter Normaldruck und bei einer Temperatur von 100 bis 150°C erfolgt.

5. Verfahren nach Anspruch 1 bis 4, dadurch gekennzeichnet, daß Essigsäure mit einer Konzentration von 50 bis 99.5 Gew% verwendet wird, wobei die Verdünnung der Essigsäure so bemessen wird, daß im phlegmatisierten Rückstand kein Wasser vorhanden ist.

6. Verfahren nach Anspruch 1 bis 5, dadurch gekennzeichnet, daß bei der Umsetzung mit Essigsäure 0.3 bis 2.0 Gew.teile Essigsäure je 1 Gew.teil Rückstand eingesetzt werden und Verweilzeiten von 0.2 bis 8.0 Stunden eingehalten werden.

7. Verfahren nach Anspruch 1 bis 6, dadurch gekennzeichnet, daß zur Auftrennung des Produkts der Umsetzung mit Essigsäure die Dünnschichtverdampfung bei einem Druck von 400 bis 500 mbar und einer Temperatur von 100 bis 180°C durchgeführt wird.

## Claims

1. Process for isolating pure diketene with recovery of materials of value by continuous, two-stage distillation of crude diketene at reduced pressure and with continuous reaction of the resulting distillation residue with acetic acid, which comprises
a) in the first distillation stage, feeding the crude diketene to a thin-film evaporator, evaporating it at reduced pressure and feeding the vapour phase to a distillation column, from the top of which pure diketene is drawn off, and combining the column bottoms, which contain a mixture of diketene and acetic anhydride, with the bottom product of the thin-film evaporator,
b) further adding, to the mixture of the bottom products from the thin-film evaporator or distillation column of the first distillation stage condensate, which is obtained in the production of ketene or the further processing thereof or pure acetic anhydride, which is obtained as bottom product in the distillation in the second distillation stage and recirculated,
c) in the second distillation stage, feeding the combined bottom products to an additional thin-film evaporator, evaporating it at reduced pressure and transferring the vapour phase to a distillation column, from the top of which a mixture containing diketene and acetic anhydride is drawn off and fed back to the distillation column of the first distillation stage, while the bottom product in the form of pure acetic anhydride is transferred out or recirculated, and
d) admixing the bottom product from the thin-film evaporator of the second distillation stage with acetic acid, feeding it to a reactor and, after the reaction has taken place, separating the reaction product in a thin-film evaporator to obtain reutilizable acetic acid, which contains acetone with or without acetic anhydride, as vapour, while an inert residue in liquid form is drawn off as bottom product.

2. Process according to Claim 1, characterized in that the thin-film evaporators and the columns of the two distillation stages are operated at a pressure of from 50 to 100 mbar and at a temperature of from 60 to 85°C.

3. Process according to Claim 1 or 2, characterized in that the thin-film evaporation in the second distillation stage is carried out in such a way that the bottom product has an acetic anhydride content of at least 15 % by weight and the bottom product in this form is immediately and continuously stabilized with acetic acid.

4. Process according to Claims 1 to 3, characterized in that the reaction with acetic acid is carried out at standard pressure and at a temperature of from 100 to 150°C.

5. Process according to Claims 1 to 4, characterized in that acetic acid having a concentration of from 50 to 99.5 % by weight is used, the dilution of the acetic acid being calculated such that no water is present in the stabilized residue.

6. Process according to Claims 1 to 5, characterized in that the reaction with acetic acid is carried out using from 0.3 to 2.0 parts by weight of acetic acid per 1 part by weight of residue and residence times are from 0.2 to 8.0 hours.

7. Process according to Claims 1 to 6, characterized in that, for separating the product of the reaction with acetic acid, the thin-film evaporation is carried out at a pressure of from 400 to 500 mbar and at a temperature of from 100 to 180°C.

## Revendications

1. Procédé pour isoler un dicétène brut, en récupérant des matières utiles, par distillation continue à deux étages de dicétènes bruts, sous pression réduite et tout en faisant réagir d'une manière continue, avec de l'acide acétique, le résidu de distillation ainsi obtenu, dans lequel :
a) dans le premier étage de distillation, on envoie le dicétène brut à un évaporateur à couche mince, on le vaporise sous pression réduite, et on envoie la phase vapeur à une colonne de distillation, en tête de laquelle on soutire du dicétène pur, et on combine au produit de queue de l'évaporateur à couche mince le fond de la colonne, contenant un mélange de dicétène et d'anhydride acétique, et
b) on introduit encore dans le mélange des produits de queue de l'évaporateur à couche mince ou de la colonne de distillation du premier étage de distillation un condensat obtenu lors de la production du cétène ou de sa post-transformation, ou encore on introduit de l'anhydride acétique pur, obtenu comme produit de queue lors de la distillation dans le deuxième étage de distillation, dans le mélange de produits de queue du premier étage de distillation, et on l'envoie en circuit fermé, et
c) dans le deuxième étage de distillation, les produits de queue ainsi réunis sont envoyés à un autre évaporateur à couche mince, on les vaporise sous pression réduite, et on envoie la phase vapeur dans une colonne de distillation en tête de laquelle on soutire un dicétène et un mélange contenant de l'anhydride acétique, lequel est renvoyé dans la colonne de distillation du premier étage de distillation, tandis que l'on soutire le produit de queue, sous forme d'anhydride acétique pur, ou on l'envoie en circuit fermé, et
d) on ajoute de l'acide acétique au résidu de l'évaporateur à couche mince du deuxième étage de distillation, on envoie ce résidu dans un réacteur et, après une réaction, on sépare le produit de la réaction dans un évaporateur à couche mince, et on obtient sous forme de vapeurs de l'acide acétique réutilisable, qui contient de l'acétone et éventuellement de l'anhydride acétique, tandis que l'on soutire sous forme d'un produit de queue un résidu inerte vis-à-vis de la réaction, sous forme liquide.

2. Procédé selon la revendication 1, caractérisé en ce que les évaporateurs à couche mince et les colonnes des deux étages de distillation sont exploités sous une pression de 50 à 100 mbar et à une température de 60 à 85°C.

3. Procédé selon les revendications 1 et 2, caractérisé en ce que l'évaporation sur couche mince dans le deuxième étage de distillation est mise en oeuvre de façon que le produit de fond ait au moins une teneur en anhydride acétique de 15 % en poids, et que le produit de fond, sous cette forme, soit immédiatement et en continu soumis à une phlegmatisation à l'acide acétique.

4. Procédé selon les revendications 1 à 3, caractérisé en ce que la réaction avec l'acide acétique s'effectue sous la pression normale et à une température de 100 à 150°C.

5. Procédé selon les revendications 1 à 4, caractérisé en ce qu'on utilise de l'acide acétique à une concentration de 50 à 99,5 % en poids, en diluant l'acide acétique de façon qu'il n'y ait pas d'eau dans le résidu phlegmatisé.

6. Procédé selon les revendications 1 à 5, caractérisé en ce que, lors de la réaction avec l'acide acétique, on utilise de 0,3 à 2,0 parties en poids d'acide acétique par parties en poids de résidu, et que l'on maintient des temps de séjour de 0,2 à 8,0 heures.

7. Procédé selon les revendications 1 à 6, caractérisé en ce que, pour séparer le produit de la réaction avec l'acide acétique, on met en oeuvre l'évaporation sur couche mince sous une pression de 400 à 500 mbar et à une température de 100 à 180°C.
